# EUROPEAN PATENT SPECIFICATION

(11) **EP 4 235 566 B1**
(45) Date of publication and mention of the grant of the patent: **18.11.2026**
(21) Application number: 22158777.7
(22) Date of filing: 25.02.2022
(51) Int. Cl.: G06T 7/00, G06T 7/11, G06T 7/33, G06T 7/30, G06T 7/174

(54) **METHOD AND SYSTEM FOR DETERMINING A CHANGE OF AN ANATOMICAL ABNORMALITY DEPICTED IN MEDICAL IMAGE DATA**
VERFAHREN UND SYSTEM ZUR BESTIMMUNG EINER VERÄNDERUNG EINER IN MEDIZINISCHEN BILDDATEN ABGEBILDETEN ANATOMISCHEN ANOMALIE
PROCÉDÉ ET SYSTÈME PERMETTANT DE DÉTERMINER UN CHANGEMENT D'UNE ANOMALIE ANATOMIQUE REPRÉSENTÉE DANS DES DONNÉES D'IMAGES MÉDICALES

(43) Date of publication of application: 30.08.2023
(73) Proprietor: Siemens Healthineers AG, 91301 Forchheim (DE)
(72) Inventor: Preuhs, Alexander, 91058 Erlangen (DE); Preuhs, Elisabeth, 91058 Erlangen (DE); Ziebandt, Valentin, 90482 Nürnberg (DE)
(74) Representative: Siemens Healthineers Patent Attorneys

(56) References cited:
- US-A1- 2010 254 584
- US-A1- 2014 313 303
- US-A1- 2021 287 799
- US-B2- 8 712 130
- ELENA BERNARDIS ET AL: "Extracting Evolving Pathologies via Spectral Clustering", 28 June 2013, INFORMATION PROCESSING IN MEDICAL IMAGING, SPRINGER BERLIN HEIDELBERG, BERLIN, HEIDELBERG, PAGE(S) 680 - 691, ISBN: 978-3-642-38867-5, XP047033341
- BARROS NETTO STELMO MAGALHÃES ET AL: "Unsupervised detection of density changes through principal component analysis for lung lesion classification", MULTIMEDIA TOOLS AND APPLICATIONS, KLUWER ACADEMIC PUBLISHERS, BOSTON, US, vol. 76, no. 18, 15 February 2017 (2017-02-15), pages 18929 - 18954, XP036297731, ISSN: 1380-7501, [retrieved on 20170215], DOI: 10.1007/S11042-017-4414-6

## Description

The present embodiments relate to medical image processing, such as image processing for x-ray images or computed tomography images.

Automated image processing for follow-up reading and longitudinal change assessment is an important task in medical imaging techniques such as computed tomography (CT) or magnetic resonance imaging (MRI). The task of recognizing changes in medical images is a technical problem due to the challenge of identifying abnormal patterns in the medical images and tracking their progression over time. For example, for a follow-up scan of a lung in COVID patients with radiographic signs of consolidation, it is important if the consolidation is getting bigger/stronger or if the lung starts to appear clearer again. Similar, for lesions which are already under treatment it is relevant if the lesion size is getting bigger, smaller, or remains the same.

Detecting pathological changes in medical images acquired at two or more time points is difficult due to the inherent complexity of the task. To begin with, abnormalities must be identified. Further, they have to be related to one another in order to infer changes from a direct comparison. What is more, changes in abnormalities are often masked or influenced by normal variations between medical images acquired at different points in time. For example, for a follow-up scan of a lung or other organ of a patient, normal anatomic changes such as respiration or other anatomical differences may mask pathological changes such as cancerous nodule growth or shrinkage. In addition, variations may stem from different image parameters such as a slightly different body regions being imaged or varying magnifications.

ELENA BERNARDIS ET AL: "Extracting Evolving Pathologies via Spectral Clustering", 28 June 2013 (2013-06-28), INFORMATION PROCESSING IN MEDICAL IMAGING, SPRINGER BERLIN HEIDELBERG, BERLIN, HEIDELBERG, PAGE(S) 680 - 691, ISBN: 978-3-642-38867-5, discloses a method for identifying pathologies in 3D+t(ime) within a spectral graph clustering framework.

BARROS NETTO STELMO MAGALHÄES ET AL: "Unsupervised detection of density changes through principal component analysis for lung lesion classification", MULTIMEDIA TOOLS AND APPLICATIONS, KLUWER ACADEMIC PUBLISHERS, BOSTON, US, vol. 76, no. 18, 15 February 2017 (2017-02-15), pages 18929-18954, ISSN: 1380-7501, DOI: 10.1007/S11042-017-4414-6, discloses a methodology for analysis, quantification and visualization of unsupervised changes in lung lesions, through principal component analysis.

US 2010 / 254 584 A1 discloses a method for assessing a tumor's response to therapy, comprising: providing images of a first study of a patient and images of a second study of the patient, the second study occurring after the first study and after the patient undergoes therapy to treat a tumor, each study comprising first and second types of functional magnetic resonance (fMR) images, performing a first registration in which the images within each study are registered, performing a second registration in which reference images from both studies are registered, segmenting the tumor in an image of each of the second registered studies; and determining that first and second fMR measure differences exist between the segmented tumor's of the first and second studies, the first fMR measure difference being obtained from the first type of fMR images, the second fMR measure difference being obtained from the second type of fMR images.

US 8,712,130 B2 discloses a method for evaluating evolution of tumoral lesions including: providing a first image of the tumoral lesions, the first image being made at a first time instant; providing a second image of the tumoral lesions, the second image being made at a second time instant that is later than the first time instant; delineating a border of the tumoral lesions in the first image and the second image; -registration of the tumoral lesions in the first image the second image; segmenting the tumoral lesions in the first image and the second image into concentric areas; quantifying changes of at least one functional parameter between the concentric areas in the first image and respective corresponding concentric areas in the second image; and visualizing the changes in a two-dimensional model of the tumoral lesions.

It is an object of the invention to provide methods and systems that allow for an improved way to determine changes in abnormalities from medical image data of a patient. In particular, it is an object of the invention to provide methods and systems that enable determining a change of an abnormality between follow-up medical image data sets of a patient taken at different instances in time.

This object is solved by a method for determining a change of an abnormality in image data of an anatomical region of a patient, a corresponding system, a corresponding computer-program product, and a computer-readable storage medium according to the independent claims. Alternative and/or preferred embodiments are object of the dependent claims.

In the following, the technical solution according to the present invention is described with respect to the claimed apparatuses as well as with respect to the claimed methods. Features, advantages or alternative embodiments described herein can likewise be assigned to other claimed objects and vice versa. In other words, claims addressing the inventive method can be improved by features described or claimed with respect to the apparatuses. In this case, e.g., functional features of the method are embodied by objective units or elements of the apparatus.

According to a first aspect, a computer-implemented method for determining a change of an abnormality in an anatomical region of a patient is provided. In other words, a method is provided for determining a change of an abnormality depicted in image data of the anatomical region of the patient. The method comprises a plurality of steps. A first step is directed to receiving a first medical image of an anatomical region of a patient, the first medical image being acquired at a first instance of time and depicting at least one abnormality in the anatomical region. A further step is directed to receiving a second medical image of the anatomical region of the patient, the second medical image being acquired at a second instance of time. A further step is directed to providing a decomposition function configured to extract, from a medical image of an anatomical region with one or more abnormalities, an abnormality image only depicting the abnormalities (the image regions of the medical image of the one or more abnormalities). A further step is directed to generating a first abnormality image of the first medical image by applying the decomposition function to the first medical image. A further step is directed to generating a second abnormality image of the second medical image by applying the decomposition function to the second medical image. A further step is directed to comparing the first abnormality image and the second abnormality image. A further step is directed to determine a change of the at least one abnormality based on the step of comparing.

In particular, the first and the second medical image can be two-dimensional images. In particular, the first and the second medical image can be three-dimensional images. In particular, the first and the second medical image can be four-dimensional images, where there are three spatial and one time-like dimensions.

In particular, the type of the medical image is related to the type of the medical imaging apparatus used for acquiring the medical image. For example, a first X-ray image and a second X-ray image are of the same type, even if they are recorded by different X-ray imaging apparatuses. In particular, the first medical image and the second medical image are of the same type if they correspond to the same anatomical region (or region of interest) in the human body. For example, a first X-ray image of a human lung and a second X-ray image of a human knee are not of the same type, even if they relate to the same patient.

In particular, the type of the medical image can be characterized by the modality used for creating the medical image and by the anatomical region that is subject of the medical image. Optionally, the type of the medical image can also be characterized by parameters (of the imaging modality) used for creating the medical image (e.g., there could be the distinction between a "low dose image" and a "high dose image").

First and second medical images may, for example, be in the form of an array of pixels or voxels. Such arrays of pixels or voxels may be representative of intensity, absorption or other parameter as a function of three-dimensional position, and may, for example, be obtained by suitable processing of measurement signals obtained by a medical imaging modality.

In particular, the first medical image and the second medical image can be medical images of the same patient.

A medical image can be identical with or encapsulated in one or more DICOM files. Whenever DICOM is mentioned herein, it shall be understood that this refers to the "Digital Imaging and Communications in Medicine" (DICOM) standard, for example according to the DICOM PS3.1 2020c standard (or any later or earlier version of said standard).

"Receiving" in the framework of the application may mean that first and second medical images are acquired from the medical imaging modalities. Further "receiving" may mean that they are acquired from an appropriate memory such as a picture archiving and communication system (PACS) or any other suitable medical image storing facility.

The first medical image may relate to an examination of the patient at a first time (first instance of time), while the second medical image may relate to an examination of the patient at a second time (second instance of time) different than the first time. The second time may be hours, days, weeks, months, or years after or before the first time. Further, there may be intervening scans or procedures between the first time and the second time.

In particular, an abnormality (another word is "abnormal structure") within a patient is an anatomical structure that differentiates said patients from other patients. In particular, an abnormality can be connected with a certain pathology of a patient.

The abnormality can be located within different organs of the patient (e.g., within the lung of a patient, or within the liver of a patient), the abnormality can also be located in between the organs of the patient. In particular, the abnormality could be a foreign body.

In particular, an abnormality can be a neoplasm (also denoted as "tumor"), in particular, a benign neoplasm, an in situ neoplasm, an malignant neoplasms and/or a neoplasms of uncertain/unknown behavior. In particular, an abnormality can be a nodule, in particular, a lung nodule. In particular, an abnormality can be a lesion, in particular, a lung lesion.

In particular, an anatomical region or object may relate to a body part of the patient. The anatomical region may comprise a plurality of anatomies and/or organs. Taking a chest image as an example, first and second medical images may show lung tissue, the rib cage, lymph nodes and others.

Changes may relate to a disease state of the patient. A change may relate to a growth, shrinkage, appearance, or disappearance of an abnormality from the first medical image to the second medical image (i.e., from the first instance of time to the second instance of time). Examples include the growth or shrinkage of nodules, the occurrence of new nodules and/or lesions and so forth.

According to some examples, the decomposition function may be based on one or more algorithms adapted to extract, from a medical image of an anatomical region with one or more abnormalities, an abnormality image only depicting the image regions of the medical image of the one or more abnormalities. The decomposition function may comprise a computer program product that, when executed on a computing unit, may control the computing unit so as to perform the task the decomposition function is configured for. The decomposition function may be provided by way of executable program code on a memory unit of the computing unit.

According to some examples, the abnormality image may have the same size as the medical image it has been extracted from (i.e., the first or second medical image). That is, it may comprise the same number of pixels or voxels as the underling medical image. In particular, the abnormality image may depict the abnormalities depicted in the underlying medical image at the same image regions (or locations) as the underlying medical image. The image regions depicting the abnormalities may also be denoted as abnormality image regions or abnormality patches. In particular, the abnormality image may comprise the pixel/voxel values of the medical image of those image regions of the medical image depicting abnormalities. In particular, the abnormality image may comprise different pixel/voxel values as the underlying medical image in image regions different from those image regions where the abnormalities are depicted. In particular, the abnormality image may comprise arbitrary pixel/voxel values in image regions different from the image regions where the abnormalities are depicted, in particular, zero or void or any constant pixel/voxel value. The abnormality image may be seen as a modified image which has been modified from the underlying medical image. In other words, the abnormality image may be seen as a synthetic image, which has been synthetically generated from the underlying medical image.

According to some examples, the method further comprises the step of providing the change to a user via a user interface. According to some examples, the step of receiving the first medical image and/or second medical image may comprise receiving a selection from the user via the user interface indicative of the first and/or second medical image.

With the proposed method, image data can directly be compared in order to derive a change in abnormalities visible in a body part of the patient. It is not required to positively identify abnormalities with a feature detection algorithm. Neither does the proposed method require to archive once detected abnormalities for a later change assessment. Moreover, the approach is highly explainable. Due to the image deompo-sition, the calculation of the corresponding change can be easily verified based on the abnormality images and is, therefore, very transparent to the user.

According to an aspect, the decomposition function is further configured to extract, from medical images of anatomical regions with one or more abnormalities, a normal image of the anatomical region not depicting the one or more abnormalities and the method further comprises the steps of generating a first normal image of the first medical image by applying the decomposition function to the first medical image, and a second normal image of the second medical image by applying the decomposition function to the second medical image.

According to some examples, the normal image may have the same size as the medical image it has been extracted from (i.e., the first or second medical image) and/or the corresponding abnormality image. That is, the normal image may comprise the same number of pixels or voxels as the underling medical image and/or the corresponding abnormality image. In particular, the normal image may not depict the abnormalities depicted in the underlying medical image. At the image regions where the underlying medical image depicts abnormalities, the normal image may instead show "normal" or "repaired" image data. In other words, the pixel/voxel values of the medical image relating to abnormalities may be altered in the normal image to depict how the image data would (likely) look like if no abnormality would be there. The normal image may be seen as a modified image which has been modified from the underlying medical image. In other words, the normal image may be seen as a synthetic normal image, which has been synthetically generated from the underlying medical image.

By also providing the normal image, a user can be provided with a notion of how the medical images would look like, if no abnormalities would be present, which may be helpful to determine if the change has been calculated correctly.

According to an aspect, the method may further comprises determining at least one image registration between the first abnormality image and the second abnormality image and the step of determining a change of the at least one abnormality is based on the at least one image registration.

Determining at least one image registration, according to some examples, may in general comprise registering a target image (e.g., the first image or the first normal image or the first abnormality image) with a reference image of a time series (e.g., the second image or the second normal image or the second abnormality image). According to some examples, this may comprise obtaining a deformation field between target and reference image that determines a relationship between the coordinate systems of the target image data and the reference image data such that each anatomical location in the target image is mapped to the same anatomical location in the reference image and vice versa. Thus, the deformation field may comprise a plurality of individual displacement vectors respectively associated with the pixels/voxels of the target image and the reference image.

According to some examples, the registration may comprise a rigid registration. A rigid registration may comprise a registration in which the coordinates of pixels/voxels in one image are subject to rotation and translation in order to register the image to another image. According to some examples, the registration may comprise and affine registration. An affine registration may comprise a registration in which the coordinates of data points in one image are subject to rotation, translation, scaling and/or shearing in order to register the image to another image. Thus, a rigid registration may be considered to be a particular type of affine registration. According to some examples, the registration may comprise a non-rigid registration. A non-rigid registration may provide different displacements for each pixel/voxel of the image to be registered and can, for example, use non-linear transformations, in which the coordinates of pixels/voxels in one image are subject to flexible deformations in order to register the image to another image. Non-linear transformations may, according to some examples, be defined using vector fields such as warp fields, or other fields or functions, defining an individual displacement for each pixel/voxel in an image. For more detailed information about image registration, reference is made to US 2011 / 0 081 066 and US 2012 / 0 235 679. Rigid image registration is very effective in cases when no anatomic change or deformations are expected. In comparison to rigid image registration, non-rigid image registration has a significantly greater flexibility as non-rigid image registrations can manage local distortions between two image sets (e.g. anatomical structure changes) but can be more complex to handle.

Basing the change-evaluation on the registration has the advantage that first and second abnormality image can be transformed into a common coordinate system. With that, it can be ensured that all abnormalities have the same scale. In turn, abnormalities can be more readily compared and artefacts in the calculation of the change are avoided.

According to some examples, the step of determining a change of the at least one abnormality based on the at least one image registration comprises aligning the first abnormality image and the second abnormality image using the registration to generate co-aligned image data, wherein the change is determined based on the co-aligned image data.

According to some examples, the step of determining a change of the at least one abnormality based on the at least one image registration comprises transforming the first abnormality image into the coordinate system of the second abnormality image or vice versa to generate transformed image data, wherein the change is determined based on the transformed image data.

According to an aspect, the at least one image registration is determined by registering the first medical image with the second medical image.

Since registrations are based on recognizing corresponding image regions based on the comprised image data, using the first and second medical image data (instead of the abnormality image) has the advantage that the image information on the basis of which the registration is performed can be increased. Accordingly, a better image registration can be provided. With that, the change determined is more accurate.

According to an aspect, the at least one image registration is determined by registering the first normal image with the second normal image.

Basing the registration on the normal images has the advantage that registration artefacts due to the abnormalities can be avoided. One reason is that there exists a number of image registration models optimized for certain major anatomies or organs, such as lung, heart, liver, kidneys, spleen, or brain while smaller structures such as abnormalities or lesions are usually not handled well be computer-assisted image registration techniques. This is because these structures are smaller, inherently more dynamic and/or more distributed. Accordingly, a better image registration can be provided. With that, the change determined is more accurate.

According to an aspect, the method further comprises calculating an deformation field based on the at least one image registration, the deformation field is suited to map the image region of the at least one abnormality in the first abnormality image to the corresponding image region of the at least one abnormality in the second abnormality image, wherein the change is determined based on the deformation field. The deformation field used in this context is subsequently also denoted as abnormality deformation field.

In other words, the change may be derived from the abnormality deformation field. This may lead to a more accurate determination of the change as compared to a separate determination of characteristics of the abnormalities (such as the size or volume of the abnormalities) and their ensuing comparison.

According to some examples, determining the change based on the abnormality deformation field may comprise calculating one or more (locally) averaged field parameters of the abnormality deformation field and determining the change based on the one or more averaged field parameters. The averaged field parameters preferably comprise at least one of: the average magnitude and the average orientation of deformation field vectors of an abnormality from the first instance of time to the second instance of time.

According to some examples, determining the change based on the abnormality deformation field may comprise determining the number of pixels mapped for an abnormality from the first instance of time to the second instance of time on the basis of the abnormality deformation field. The more pixels are mapped to one pixel in the second image, the smaller is the growth and vice versa.

According to some examples, the abnormality deformation field is calculated based on a non-rigid image registration.

According to some examples, the step of determining a change of the at least one abnormality based on the at least one image registration comprises transforming the first abnormality image into the coordinate system of the second abnormality image or vice versa to generate transformed image data, and determining the abnormality deformation field is based on the transformed image data. Thus, in other words, the generation of the abnormality deformation field may be seen as an outcome of a second image registration between first and second abnormality images after having been brought into the same coordinate system by the (first) image registration.

According to an aspect, the step of determining a change comprises calculating a score measuring the size change of the at least one abnormality from the first instance of time to the second instance of time.

With that, a progression of a pathology can directly be quantified which is of high importance for coming to the right treatment decisions.

According to an aspect, the decomposition function comprises an inpainting function configured to inpaint abnormalities within a medical image to generate a normal image of the medical image, wherein the normal image does not depict any abnormalities. The decomposition function is further configured to extract the abnormality image from the medical image by subtracting the normal image from the medical image or vice versa.

In general, in the field of imaging, the term inpainting denotes a process where missing or defective or simply unwanted parts of an image are filed-in to create a synthetic image (without the parts to be corrected). In general, inpainting of images can be done manually or automatically, in particular, by image processing algorithms. In particular, automatic inpainting can utilize information within the images outside the parts to be corrected to infer about suited "replacement content" of the parts to be corrected. In the framework of this application the "parts to be corrected" may be equated with the abnormalities and the synthetic image would be the normal image.

Inpainting algorithms can be based on structural and/or textural aspects of images. Furthermore, inpainting algorithms can be classical or learning-based inpainting algorithms. In particular, inpainting methods can also take into account external data not contained in the image (non-local algorithms). For further information, reference is made to Ber-talmio, Marcelo & Sapiro, Guillermo & Caselles, Vicent & Bal-lester, C., "Image inpainting", Proceedings of the ACM SIGGRAPH Conference on Computer Graphics, 417-424, (2000).

By relying on inpainting functions, normal and abnormal image components of medical images can be readily be decomposed which allows for a swift comparison of abnormalities for follow-up reading of medical images.

According to an aspect, the decomposition function comprises a trained function. According to some examples, the trained function may, in particular, comprise a conditional Generative Adversarial Network (cGAN).

In general, a trained function mimics cognitive functions that humans associate with other human minds. In particular, by training based on training data, the trained function is able to adapt to new circumstances and to detect and extrapolate patterns.

In general, parameters of a trained function can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained functions can be adapted iteratively by several steps of training.

In particular, a trained function can comprise a neural network, a support vector machine, a decision tree and/or a Bayesian network, and/or the trained function can be based on k-means clustering, Qlearning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

A generative adversarial network or function comprises a generator part or function and a classifier or discriminator part or function. According to some examples, the generator creates a normal image from a medical image comprising abnormalities and the discriminator distinguishes between synthetically created normal images and real normal images. The training of the generator and/or of the discriminator is based, in particular, on the minimization of a cost function in each case. According to some examples, the cost function is referred to as adversarial loss. The cost function can be minimized, in particular, by back propagation. If the generator and the discriminator are given by a network, in particular by an artificial neural network, then the GA algorithm is also referred to as GA networks (also "GAN", which is an acronym for "generative adversarial networks". These are known in particular from the publication by Ian J. Goodfellow, "Generative Adversarial Networks", arxiv 1406.2661 (2014).

Conditional generative adversarial functions or networks additionally make use of labels to control the output of the generator. With that, the conditional generation of images by the generator can be fostered. Image generation can be conditional on a class label, if available, allowing the targeted generated of images of a given type. According to some examples, the class labels may involve a normal label for images not comprising abnormalities provided during training or an abnormal label for images comprising abnormalities provided during training.

The usage of trained functions in general has the advantage that a more comprehensive and faster screening of the available information can be made. In this regard, trained learned functions may identify abnormalities in the available data that are not accessible for a human. What is achieved, in particular, with generative adversarial algorithms or networks by a training of the generator and/or of the discriminator is that, on the one hand, the generator manages to create synthetic data, which is so good that that the discriminator (incorrectly) classifies it as real. On the other hand, the discriminator is optimized to distinguish as well as possible between real data and synthetic data. In games theory a generative adversarial network can also be interpreted as a zero-sum game. The usage of conditional generative adversarial networks enables the targeted generation of synthetic normal image data. Moreover, it can lead to better performing trained functions in the form of more stable training, faster training, and/or generated normal images that have better quality.

According to some examples, the trained function (the conditional generative adversarial network) has been trained based on real normal images and/or real abnormal images, the real normal images depicting an anatomical region of a patient not comprising any abnormality in the anatomical region and the real abnormalities images depicting only abnormalities in an anatomical region of a patient.

According to some examples, the trained function (the conditional generative adversarial network) has been trained by providing training data with labels, the labels at least indicating normal images not comprising abnormalities.

According to some examples, the conditional generative adversarial network has been trained based on a first loss function implemented as a feedback to the generator, the first loss function measuring the quality of the resulting normal image. In particular, the first loss function is implemented as a feedback from the discriminator to the generator (adversarial loss).

According to some examples, the conditional generative adversarial network has been trained based on a second loss function implemented as a feedback to the generator, the second loss function measuring the quality of the resulting abnormality image. In particular, the second loss function may be based on a comparison with verified (i.e., ground truth) abnormality images obtained from the image data and/or on a measure to qualify the structure of the abnormalities, in particular, a sparsity loss.

According to some examples, the sparsity loss is based on a weighting of the contribution of pixel or voxel intensities to a loss function based on their spatial distance to a pixel or voxel with known intensity. In particular, the weighting can be an exponential function of the spatial distance.

The usage of the second loss function in general has the advantage that a two-fold adaptation and optimization of the trained function can be reached. In turn, this may improve the performance of the method in follow-up reading situations.

According to another aspect, the method further comprises providing an assistance image based on the first and/or second medical image with the at least one abnormality and/or the change being highlighted.

The assistance image may comprise a rendering of the first and/or second medical image with the change highlighted. The rendering may rely on known rendering procedures, such as ray-casting, ray-tracing, texture-rendering, image projections or the like. The term "highlighted" in this context may mean that the changes are visually enhanced in brightness, color, and/or intensity. In addition to that or as an alternative, the changes may be highlighted using symbols. The highlighting may be effected based on information as to the changes, such as position, volume and amount of change. Highlighting may furthermore mean using a heatmap wherein, e.g., the amount of change is color-coded. For instance, shrinking nodules may be assigned a different color than growing nodules and/or new nodules. The highlighting may be visualized as an overlay image on the first and/or second medical image.

By providing a rendering of the submitted image data set with the change highlighted, the user can immediately infer what changes happened and where these changes occurred. This helps guiding the image reading and therefore increases the usability of the method and provides an improved assistance to the user for deriving a medical diagnosis.

According to an aspect, the anatomical region comprises the lung of the patient and the at least one abnormality comprises a lung lesion in the lung of the patient, the lung lesion in particular comprising any one of: a lung nodule, a consolidation, or an emphysema.

By taking lung tissue and corresponding abnormalities into account, the user is provided with assistance for judging the progression of pathologies of high clinical relevance.

According to an aspect, first and second medical images are X-ray images of the chest of the patient.

X-ray images are well suited to determine changes, due to the fact that they are widely used. Thereby, the image projection made in an X-ray scan allows to screen broad regions of interest. Further, the two-dimensionality of X-ray images enables the swift application of the method steps at comparable low computational costs.

According to an aspect, the step of comparing the first abnormality image and the second abnormality image comprises matching a representation of the at least one abnormality in the first abnormality image with a representation of the at least one abnormality in the second abnormality image (optionally on the basis of the registration and/or on the basis of the second registration and/or on the basis of the abnormality deformation field). According to some examples, the step of determining a change of the at least one abnormality is based on the step of matching.

The matching may be conceived as a step of identifying pairs of associated abnormality representations or patches in the first and second abnormality images. In an example embodiment, for each pair a probability may be calculated that the two representations describe the same abnormality, for example taking into account the proximity of transformed (aligned) locations, whether they are of the same type and how similar other parameters are. Abnormality patches of one abnormality image which cannot be matched with corresponding patches in the respective other abnormality image may relate to newly occurred or vanished abnormalities.

According to an aspect, the first and second abnormality images and/or the first and second normal images are generated in parallel (that is, not sequentially). This has the advantage of faster processing.

According to an aspect, a system for determining a change of an abnormality in an anatomical region of a patient is provided. The system comprises an interface unit and a computing unit. The interface unit is configured to receive a first medical image of an anatomical region of a patient, the first medical image being acquired at a first instance of time and depicting at least one abnormality in the anatomical region, and to receive a second medical image of the anatomical region of the patient, the second medical image being acquired at a second instance of time. The computing unit is configured to provide a decomposition function configured to extract, from a medical image of an anatomical region with one or more abnormalities, an abnormality image only depicting the image regions of the medical image of the one or more abnormalities. The computing unit is further configured to generate a first abnormality image of the first medical image by applying the decomposition function to the first medical image. The computing unit is further configured to generate a second abnormality image of the second medical image by applying the decomposition function to the second medical image. The computing unit is further configured to compare the first abnormality image and the second abnormality image. The computing unit is further configured to determine a change of the at least one abnormality based on the step of comparing.

The computing unit may comprise an image decomposition unit configured to host, run and/or apply the decomposition function. The computing unit may comprise an image registration unit configured to generate at least one image registration. Optionally, the registration unit may further be configured to generate one or more deformation fields as an outcome of the image registration. The computing unit may comprise a comparator unit for comparing medical images (in particular, abnormality images) and for determining a change of an abnormality. Optionally, the computing unit may further comprise a visualization unit configured to generate a visualization (for a user) highlighting the identified changes.

The computing unit may be realized as a data processing system or as a part of a data processing system. Such a data processing system can, for example, comprise a cloud-computing system, a computer network, a computer, a tablet computer, a smartphone and/or the like. The computing unit can comprise hardware and/or software. The hardware can comprise, for example, one or more processor, one or more memories and combinations thereof. The one or more memories may store instructions for carrying out the method steps according to the invention. The hardware can be configurable by the software and/or be operable by the software. Generally, all units, sub-units or modules may at least temporarily be in data exchange with each other, e.g., via a network connection or respective interfaces. Consequently, individual units may be located apart from each other.

The interface unit may comprise an interface for data exchange with a local server or a central web server via internet connection for receiving the reference image data or follow-up image data. The interface unit may be further adapted to interface with one or more users of the system, e.g., by displaying the result of the processing by the computing unit to the user (e.g., in a graphical user interface) or by allowing the user to adjust parameters for image processing or visualization and/or to select first and/or second medical images.

According to other aspects, the invention further relates to an image analysis system comprising the above system and a medical image system (or medical information system) configured to acquire, store and/or forward at least first and second medical images. Thereby, the interface unit is configured to receive the first and second medical images form the medical image system.

According to some examples, the medical image system comprises one or more archive stations for storing first and second medical image data sets which may be realized as a cloud storage or as a local or spread storage, e.g., as a PACS (Picture Archiving and Communication System). Further, the medical image system may comprise one or more medical imaging modalities, such as a computed tomography system, a magnetic resonance system, an angiography (or C-arm X-ray) system, a positron-emission tomography system, a mammography system, or the like.

According to other aspects, the systems are adapted to implement the inventive method in their various aspects for determining a change of an abnormality in an anatomical region of a patient. The advantages described in connection with the method aspects may also be realized by the correspondingly configured systems' components.

According to another aspect, the present invention is directed to a computer program product comprising program elements which induce a computing unit of a system for determining a change of an abnormality in an anatomical region of a patient to perform the steps according to one or more of the above method aspects, when the program elements are loaded into a memory of the computing unit.

According to another aspect, the present invention is directed to a computer-readable medium on which program elements are stored that are readable and executable by a computing unit of a system for determining a change of an abnormality in an anatomical region of a patient according to one or more method aspects, when the program elements are executed by the computing unit.

The realization of the invention by a computer program product and/or a computer-readable medium has the advantage that already existing providing systems can be easily adapted by software updates in order to work as proposed by the invention.

The computer program product can be, for example, a computer program or comprise another element next to the computer program as such. This other element can be hardware, e.g., a memory device, on which the computer program is stored, a hardware key for using the computer program and the like, and/or software, e.g., a documentation or a software key for using the computer program. The computer program product may further comprise development material, a runtime system and/or databases or libraries. The computer program product may be distributed among several computer instances.

### Brief description of the drawings

Characteristics, features and advantages of the above-described invention, as well as the manner they are achieved, become clearer and more understandable in the light of the following description of embodiments, which will be described in detail with respect to the figures. This following description does not limit the invention on the contained embodiments. Same components, parts or steps can be labeled with the same reference signs in different figures. In general, the figures are not drawn to scale. In the following:
FIG. 1 schematically depicts an embodiment of a system for determining a change of an abnormality in an anatomical region of a patient;
FIG. 2 schematically depicts a method for determining a change of an abnormality in an anatomical region of a patient according to an embodiment;
FIG. 3 schematically depicts method steps for determining a change of an abnormality in an anatomical region of a patient according to an embodiment;
FIG. 4 schematically depicts method steps for determining a change of an abnormality in an anatomical region of a patient according to an embodiment;
FIG. 5 schematically depicts an exemplary data flow diagram in connection with a method for determining a change of an abnormality in an anatomical region of a patient according to an embodiment;
FIG. 6 schematically depicts a method for providing a trained function for decomposing medical images according to an embodiment;
FIG. 7 schematically depicts an exemplary data flow diagram in connection with a method for providing a trained function according to an embodiment; and
FIG. 8 schematically depicts a system for providing a trained function.

### Detailed description of embodiments

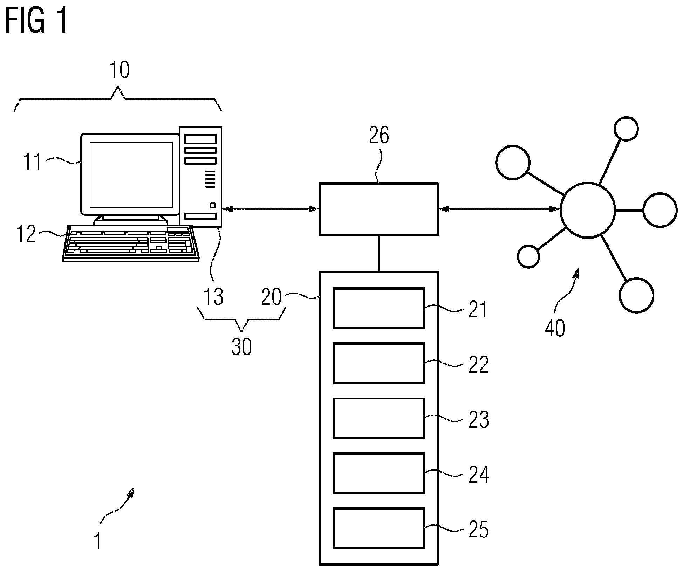

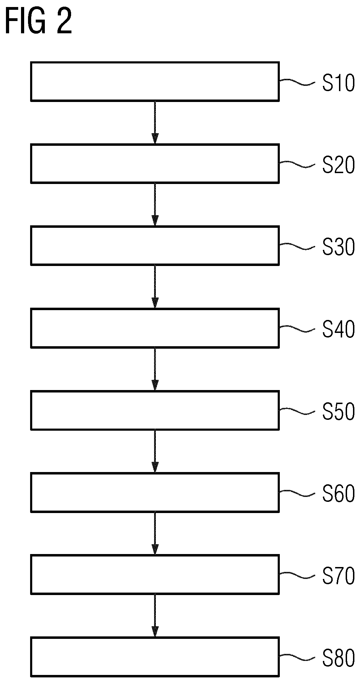

**Figure 1** depicts a system 1 for determining a change CA of an abnormality A in an anatomical region of a patient. In this regard, system 1 is adapted to perform the methods according to one or more embodiments, e.g., as further described with reference to Figures 2 to 5. A user of system 1, according to some examples, may generally relate to a healthcare professional such as a physician, clinician, technician, radiologist, pathologist and so forth.

System 1 comprises a user interface 10 (as part of the interface unit) and a processing system 20 (as part of the computing unit 30). Further, system 1 may comprise or be connected to a medical information system 40. The medical information system 40 may generally be configured for acquiring and/or storing and/or forwarding first and second medical images IM1, IM2. For instance, medical information system 40 may comprise one or more archive/review station (not shown) for storing first and second medical images IM1, IM2. The archive/review stations may be embodied by one or more databases. In particular, the archive/review stations may be realized in the form of one or more cloud storage modules. Alternatively, the archive/review stations may be realized as a local or spread storage, e.g., as a PACS (Picture Archiving and Communication System). According to some examples, medical information system 40 may also comprise one or more medical imaging modalities (not shown), such as a computed tomography system, a magnetic resonance system, an angiography (or C-arm X-ray) system, a positron-emission tomography system, a mammography system, an X-ray system, or the like.

First and second medical images IM1, IM2 may be three-dimensional image data sets acquired, for instance, using an X-ray system, a computed tomography system or a magnetic resonance imaging system or other systems. The image information may be encoded in a three-dimensional array of m times n times p voxels. First and second medical images IM1, IM2 may include a plurality of image slices which are stacked in a stacking direction to span the image volume covered by the respective first and second medical images IM1, IM2.

Further, first and second medical images IM1, IM2 may comprise two-dimensional medical image data with the image information being encoded in an array of m times n pixels. According to some examples, these two-dimensional medical images may have been extracted from three-dimensional medical image data sets.

An ensemble of voxels or pixels may be designated as image data of the respective data set in the following. In general, any kind of imaging modalities and scanners may be used for acquiring such image data. Generally, first and second medical images IM1, IM2 show a body part or an anatomical region or an anatomic object of a patient which may comprise various anatomies and organs. Considering the chest area as a body part, first and second medical images IM1, IM2 might, for instance, depict the lung lobes, the rib cage, the heart, lymph nodes, and so forth.

While one of the first and second medical images IM1, IM2 (either the first medical image IM1 or the second medical image IM2) has been taken at an earlier examination at a first time, the respective other relates to a follow-up examination at a later stage at a second time. The second time may be hours, days, weeks, months, or years after the first time. Further, there may be intervening scans or procedures between the first time and the second time. In an embodiment, the medical image data sets have been acquired using the same or similar settings and parameters. Similar settings and parameters may include, for example, the same medical imaging modality, a similar dose (if available), the same phase timing, x-ray source voltage, contrast agent, MRI-protocol, and the like. Alternatively, the image data sets - despite of the fact that they depict the same body part - may have been acquired using different imaging modalities and/or different settings for the imaging modalities.

First and second medical images IM1, IM2 may be formatted according to the DICOM format. DICOM (=Digital Imaging and Communications in Medicine) is an open standard for the communication and management of medical imaging information and related data in healthcare informatics. DICOM may be used for storing and transmitting medical images and associated information enabling the integration of medical imaging devices such as scanners, servers, workstations, printers, network hardware, and picture archiving and communication systems (PACS). It is widely adopted by clinical syndicates, hospitals, as well as for smaller applications like doctors' offices or practices. A DICOM data object consists of a number of attributes, including items such as patient's name, ID, etc., and also special attributes containing the image pixel data and metadata extracted from the image data.

User interface 10 comprises a display unit 11 and an input unit 12. User interface 10 may be embodied by a mobile device such as a smartphone or tablet computer. Further, user interface 10 may be embodied as a workstation in the form of a desktop PC or laptop. Input unit 12 may be integrated in display unit 11, e.g., in the form of a touch screen. As an alternative or in addition to that, input unit 12 may comprise a keyboard, a mouse or a digital pen and any combination thereof. Display unit 11 may be configured for displaying the first and second medical images IM1, IM2 and any results and images derived therefrom in the course of the method execution such as the assistance image AI and the change CA.

User interface 10 further comprises an interface computing unit 13 configured to execute at least one software component for serving display unit 11 and input unit 12 in order to provide a graphical user interface for allowing the user to select a target patient's case to be reviewed. In addition, interface computing unit 13 may be configured to communicate with medical information system 40 or processing system 20 for receiving first and second medical images IM1, IM2. The user may activate the software component via user interface 10 and may acquire the software component, e.g., by downloading it from an internet application store. According to an example, the software component may also be a client-server computer program in the form of a web application running in a web browser. The interface computing unit 13 may be a general processor, central processing unit, control processor, graphics processing unit, digital signal processor, three-dimensional rendering processor, image processor, application specific integrated circuit, field programmable gate array, digital circuit, analog circuit, combinations thereof, or other now known device for processing image data. User interface 10 may also be embodied as a client.

Processing system 20 may comprise sub-units 21-25 configured to process the first and second medical images IM1, IM2, in order to determine a change CA of at least an abnormality A between the first medical image IM1 and the second medical image IM2, and, optionally, to provide a visualization of the change CA, e.g., in the form of an assistance image AI.

Processing system 20 may be a processor. The processor may be a general processor, central processing unit, control processor, graphics processing unit, digital signal processor, three-dimensional rendering processor, image processor, application specific integrated circuit, field programmable gate array, digital circuit, analog circuit, combinations thereof, or other now known device for processing image data. The processor may be single device or multiple devices operating in serial, parallel, or separately. The processor may be a main processor of a computer, such as a laptop or desktop computer, or may be a processor for handling some tasks in a larger system, such as in the medical information system or the server. The processor is configured by instructions, design, hardware, and/or software to perform the steps discussed herein. Alternatively, processing system 20 may comprise a real or virtual group of computers like a so called 'cluster' or 'cloud'. Such server system may be a central server, e.g., a cloud server, or a local server, e.g., located on a hospital or radiology site. Further, processing system 20 may comprise a memory such as a RAM for temporally loading first and second medical images IM1, IM2. Alternatively, such memory may as well be comprised in user interface 10.

Sub-unit 21 is a data retrieval module or unit. It is configured to access and search the medical information system 40 for first and second medical images IM1, IM2. For instance, sub-unit 21 may configured to retrieve a second medical image IM2 in connection with a first medical image IM1. Specifically, sub-unit 21 may be configured to formulate search queries and parse them to medical information system 40.

Sub-unit 22 can be conceived as an image decomposition module or unit. It is configured to process first and second medical images IM1, IM2 in order to respectively decompose first and second medical images IM1, IM2 into a normal medical image N-IM1, N-IM2 not depicting any of the abnormalities A comprised in first and second medical images IM1, IM2, and an abnormality image A-IM1, A-IM2 only depicting the abnormalities A comprised in first and second medical images IM1, IM2. In particular, sub-unit 22 may be configured to replace any image data relating to abnormalities A in the first and second medical images IM1, IM2 by synthetic image data not depicting abnormalities A. To this end, sub-unit 22 may be configured to run an accordingly configured image processing function in the form a decomposition function TF.

Sub-unit 23 may be conceived as a registration module or unit. Sub-unit 23 may configured to perform a registration IR1 of the first medical image IM1 and the second medical image IM2. Sub-unit 23 may further be configured to perform a registration IR1 of the first normal medical image N-IM1 and the second normal medical image N-IM2. Sub-unit 23 may further be configured to perform a second registration IR2 of the second abnormality image A-IM2 and the transformed first abnormality image A-IM1-T, wherein the transformed first abnormality image A-IM1-T has been transformed on the basis of registration IR1. Of note, the transformation of the first abnormality image A-IM1 is only meant as an example. Likewise the second abnormality image A-IM2 can be transformed on the basis of registration IR1. The ensuing second registration IR2 could then be based on the first abnormality image A-IM1 and a transformed second abnormality image which has been transformed on the basis of registration IR1. In other words, providing registration IR1 has the goal to provide an image registration on the basis of which one abnormality image AIM1, A-IM2 image can be transformed into the coordinate system of the respective other abnormality image A-IM2, A-IM1. Sub-unit 23 may further be configured to calculate a coordinate transformation which essentially converts the image data of one image into the coordinate system of the other image. The calculation result provided by sub-unit 23 may be in the form of a two or three-dimensional transformation matrix or deformation field DF1, DF2. Sub-unit 23 may be configured to apply one or more image registration techniques comprising rigid image registrations, affine image registrations, non-rigid image registrations and any combination thereof. To improve the result of the registration, sub-unit 23 may optionally be configured to mathematically fit the calculation result to one or more motion models for soft tissue deformation.

Sub-unit 24 may be configured as a comparator module or unit. Sub-unit 24 may be configured to correlate different representations of an abnormality A with one another. In particular, sub-unit 24 may be configured to do this on the basis of the abnormality images A-IM1, A-IM2, transformed abnormality images A-IM1-T and the registrations IR1 and IR2. Further sub-unit 24 may be configured to quantify a change CA of an abnormality A on the basis of the correlation. To this end, sub-unit 24 may be configured to determine a size and/or volume and/or intensity and/or texture and/or other parameter change of an abnormality A from the first medical image IM1 to the second medical image IM2. Further, sub-unit 24 may configured to derive the change CA from an evaluation of the deformation field DF2 associated with the second registration IR2 (also denoted as abnormality deformation field DF2).

Sub-unit 25 is a visualization module configured to translate or convert the determined change CA as identified by sub-unit 24 into a suitable representation for displaying to the user. The suitable representation can be in the form of an assistance image AI in which the change CA is visually encoded. This may mean that the change CA is enhanced in the visualization. Specifically, sub-unit 25 may be configured to run or execute an algorithm for rendering a semi-transparent overlay image from the change CA to be superimposed over correspondingly rendered first or second medical images IM1, IM2. Moreover, sub-unit 25 may be configured to highlight the change CA in the form of symbols or labels in the first and/or second medical image IM1, IM2.

The designation of the distinct sub-units 21-25 is to be construed by way of example and not as limitation. Accordingly, sub-units 21-25 may be integrated to form one single unit (e.g., in the form of "the computing unit 30") or can be embodied by computer code segments configured to execute the corresponding method steps running on a processor or the like of processing system 20. The same holds true with respect to interface computing unit 13. Each sub-unit 21-25 and interface computing unit 13 may be individually connected to other sub-units and or other components of the system 1 where data exchange is needed to perform the method steps. For example, sub-units 21 and 25 may be connected via an interface 26 to medical information system 40 for retrieving medical images IM1, IM2. Likewise, interface 26 may connect the sub-units 21 to 25 to interface computing unit 13 for forwarding the results of the computation to the user and collect user inputs.

Processing system 20 and interface computing unit 13 together may constitute the computing unit 30. Of note, the layout of computing unit 30, i.e., the physical distribution of interface computing unit 13 and sub-units 21-25 is, in principle, arbitrary. For instance, sub-unit 25 (or individual elements of it or specific algorithm sequences) may likewise be localized in user interface 10. The same holds true for the other sub-units 21-25. Specifically, processing system 20 may also be integrated in user interface 10. As already mentioned, processing system 20 may alternatively be embodied as a server system, e.g., a cloud server, or a local server, e.g., located on a hospital or radiology site. According to such implementation, user interface 10 could be designated as "frontend" or "client" facing the user, while processing system 20 could then be conceived as "backend" or server. Communication between user interface 10 and processing system 20 may be carried out using the https-protocol, for instance. The computational power of the system may be distributed between the server and the client (i.e., user interface 10). In a "thin client" system, the majority of the computational capabilities exists at the server. In a "thick client" system, more of the computational capabilities, and possibly data, exist on the client.

Individual components of system 1 may be at least temporarily connected to each other for data transfer and/or exchange. User interface 10 communicates with processing system 20 via interface 26 to exchange, e.g., medical images IM1, IM2, N-IM1, N-IM2, A-IM1, A-IM2, or the result CA of the computation. For example, processing system 20 may be activated on a request-base, wherein the request is sent by user interface 10. Further, processing system 20 may communicate with medical information system 40 in order to retrieve a target patient's case. As an alternative or in addition to that, user interface 10 may communicate with medical information system 40 directly. Medical information system 40 may likewise be activated on a request-base, wherein the request is sent by processing system 20 and/or user interface 10. Interface 26 for data exchange may be realized as hardware- or software-interface, e.g., a PCI-bus, USB or fire-wire. Data transfer may be realized using a network connection. The network may be realized as local area network (LAN), e.g., an intranet or a wide area network (WAN). Network connection is preferably wireless, e.g., as wireless LAN (WLAN or Wi-Fi). Further, the network may comprise a combination of different network examples. Interface 26 for data exchange together with the components for interfacing with the user 11, 12 may be regarded as constituting an interface unit of system 1.

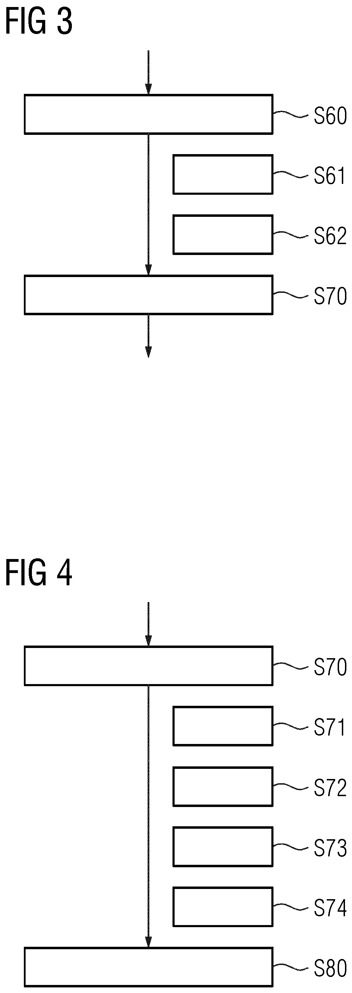

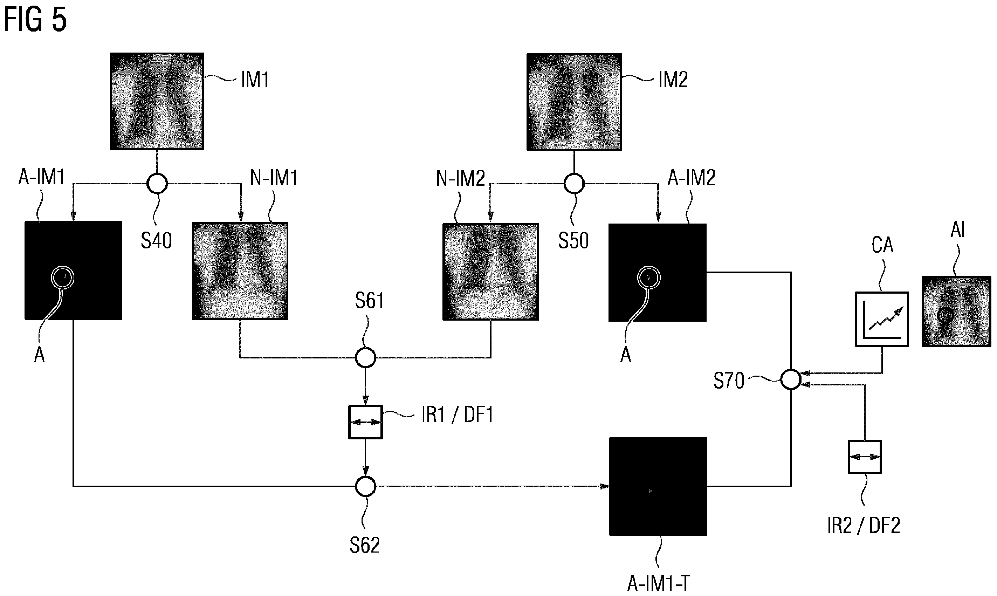

**Figure 2** depicts a method for determining a change CA of an abnormality A in an anatomical region of a patient according to an embodiment. Additional optional sub-steps according to some embodiments are shown in Figures 3 and 4. Corresponding data streams are illustrated in Figure 5. The method comprises several steps. The order of the steps does not necessarily correspond to the numbering of the steps but may also vary between different embodiments of the present invention. Further, individual steps or a sequence of steps may be repeated.

In a first step S10, the first medical image IM1 is received. The first medical image IM1 can be seen as the target image on the basis of which a user wants to perform a follow-up analysis. This may involve selecting the first medical image IM1 from a plurality of cases, e.g., stored in the medical information system 40. The selection may be performed manually by a user, e.g., by selecting appropriate image data in a graphical user interface running in the user interface 10. Alternatively, the first medical image IM1 may be provided to the computing unit 30 by a user by way of uploading the image data set IM to the computing unit 30.

A second step S20 is directed to retrieving at least one second medical image IM2 corresponding to the first medical image IM1 from the medical information system 40. To this end, the first medical image IM1 may be read in order to extract information from the first medical image IM1 on the basis of which the medical information system 40 can be queried for suitable second medical images IM2 of the patient. This information may include, data identifiers, e.g., in the form of an accession number or a patient ID, information indicative of a patient, case and/or disease type, the type of medical image data set (2D, 3D, MR-data, CT-data, etc.), imaging modality and imaging parameters used, the point in time the image data set was acquired, treatments administrated to the patient, and so forth. This information may be read from the (DICOM)-header or the body of the first and second medical images IM1, IM2. As an alternative, all or part of this information may be supplemented by the user upon upload.

Step S30 is directed to provide a decomposition function TF which is an image processing function configured to decompose a medical image IM1, IM2 into a normal image N-IM1, N-IM2 and an abnormality image A-IM1, A-IM2. Exemplary embodiments of the decomposition function TF will be given in connection with Figures 5 to 7.

Step S40 is an image processing step which is directed to decompose the first medical image IM1 into a first normal image N-IM1 and a first abnormality image A-IM1 by applying the decomposition function TF to the image data of the first medical image IM1. According to some examples, step S40 comprises generating the first normal image N-IM1 by applying the decomposition function TF on the first medical image IM1 and generating the first abnormality image A-IM1 by subtracting the first normal image N-IM1 from the first medical image IM1 (or vice versa).

Step S50 is an image processing step directed to decompose the second medical image IM2 into a second normal image N-IM2 and a second abnormality image A-IM2 by applying the decomposition function TF to the image data of the second medical image IM2. Apart from the fact that step S50 is directed to decompose the second medical image IM2, step S50 may substantially correspond to step S40. Steps S40 and S50 may be executed in parallel.

At step S60, the first and second abnormality images A-IM1, A-IM2 are compared to one another. This may involve finding (optional sub-step S61) a registration IR1 between the image spaces of first and second images IM1, IM2 to define a common coordinate system, and transforming (optional sub-step S62) the first and/or second abnormality image A-IM1, A-IM2 such that the image data therein comprised has a common coordinate system.

At step S70, a change CA of at least one abnormality A is determined based on the processing of step S60. This may involve correlating different representations of an abnormality A in first and second medical images IM1, IM2 with one another (optional sub-step S71), determining a second registration IR2 between the first and second abnormality images AIM1, A-IM2 transformed into a common coordinate system (optional sub-step S72), and quantifying the change CA (optional sub-step S73).

At optional step S80, the quantified change CA in medical findings is used to generate a further result. The result may be in the form of a viewable result for a user, i.e., in a human readable format. As such, the result may be in the form of a structured report in which the change CA in the at least one abnormality A is indicated. For instance, the structured report may be in the form of a radiology report prefilled by the system 1 with the determined change CA. Further, the result generated in step S80 may be in the form of an assistance image AI. Generating the assistance image AI may comprise rendering one or more representations of the first and/or second medical image IM1, IM2 with the change CA highlighted for the user, e.g., by introducing symbols or numbers in the vicinity of the abnormalities A, applying color maps or heatmaps, and/or adjusting brightness or luminescence values of the rendering, in particular, in order to indicate to the user where the change CA occurred and/or what magnitude it has. The rendering may be a two-dimensional rendering on the basis of an appropriate representation of the first and second medical images IM1, IM2 such as a cross-section or slice through the image volume. Moreover, the result may be provided in the form of a table or a trending graph on the basis of the change CA. Of note, the result may not only reflect the comparison of the first medical image IM1 with one second medical image IM2 but with a plurality of second medical images IM2.

In **Figure 3****,** an optional configuration of step S60 is schematically shown. In sub-step S61 a registration IR1 is obtained which links the coordinate systems of the first medical image IM1 and the second medical image IM2. In other words, a transformation is calculated which is capable of transforming the respective image data of one medical image IM1, IM2 into the coordinate system of the respective other IM2, IM1. The registration IR1 may be based on the first and second medical images IM1, IM2 as such or on the first and second normal images N-IM1, N-IM2. In step S61, at least part of the first medical image IM1 (or first normal image N-IM1) is registered with at least part of the second medical image IM2 (or second normal image N-IM2). Essentially, this may comprise identifying corresponding data points in the two images. Having identified such corresponding data points, it is possible to calculate the local offset between these corresponding points which provides an indication of the local shift in coordinate systems between the two images. Doing this for a plurality of corresponding data points sufficiently distributed in the underlying image volumes already provides a good indication of the displacements and deformations between the respective image data. To appropriately aggregate these individual contributions into a coherent two or three-dimensional transformation function or deformation field DF1, various registration techniques may be used. These techniques may comprise rigid registrations, affine registrations, non-rigid registrations, non-affine registrations and any combination thereof.

At sub-step S62, the registration IR1 or rather the deformation field DF1 is used to transform the first and second abnormality images A-IM1, A-IM2 into a common coordinate system. In particular, the first abnormality image A-IM1 may be transformed into the coordinate system of the second abnormality image A-IM2 to generate a transformed first abnormality image T-A-IM1 or vice versa.

In **Figure 4****,** an optional configuration of step S70 is schematically shown. Once the abnormality images A-IM1, A-IM2 have been processed according to step S60, they are in principle in shape that the abnormalities A depicted therein can be compared and a change CA can be quantified.

In sub-step S71, the different representations of an abnormality A in first and second medical images IM1, IM2 may be correlated. For instance, a probability may be calculated that image patches depicting an abnormality in the first and second abnormality images A-IM1, A-IM2 relate to the same abnormality, for example taking into account the proximity of transformed (aligned) locations of abnormality image data, whether they are of the same morphology, and how similar other parameters are.

In sub-step S72, a second registration IR2 may be determined and a corresponding deformation field DF2 (abnormality deformation field) may calculated that is suited to map abnormality representations in the first abnormality image AIM1 to corresponding abnormality representations in the second abnormality image A-IM1. Here, essentially the same processing can be applied as explained in connection with step S61. In particular, step S72 may employ a non-rigid registration.

In sub-step S73, the change CA is quantified, and a disease progression score may be determined on that basis.

According to some examples, the change CA may be based on one or more different observables. According to some examples, one or more of these observables may be based on the evaluation of the deformation field DF2. For instance, one or more observables may be based on average vector field properties of the deformation field DF2 per abnormality A. For example, one or more observables may comprise an average magnitude and/or an average orientation of the vectors comprised in the deformation field DF2 for the at least one abnormality A. According to some examples, one or more of the observables may be based on a number of respective pixels/voxels mapped from a representation of the at least one abnormality A in the first abnormality image A-IM1 to a representation of the at least one abnormality A in the second abnormality image A-IM2 based on the second registration IR2 and/or the deformation field DF2. According to some examples, one or more of the observables may be based on a change of one or more size-related parameters of the at least one abnormality A from the first instance in time to the second, such as a diameter, a surface, or a volume. According to some examples, one or more of the observables may be based on a change of one or more attribute-related parameters of the at least one abnormality A from the first instance in time to the second, such as an image pattern, an image intensity, a boundary property (e.g., smoothness or a degree of spiculation). In particular, the attribute-related parameters may be extracted from the image data of the representations of the at least one abnormality A in the first and second abnormality images A-IM1, A-IM2.

Based on the change CA, a disease progression score may be calculated. The disease progression score may, in particular, be based on one or more observables. Specifically, the disease progression score may be based on two or more different observables. According to some examples, a mapping of the determined change CA into a suitable disease progression score may be performed by a learned network, which is trained using expert rating of disease progression.

An overview of one embodiment of the method is provided in **Figure 5****.** Instead of using a single-time-point measure which is compared for different images IM1, IM2 acquired at different stages, respective imaging data is directly used as input (i.e., two images acquired at different timepoints). First, a material decomposition of the two images is performed in steps S40 and S50. These material decomposition steps S40 and S50 respectively separate first and second images IM1, IM2 into "normal" images N-IM1, N-IM2 (i.e., without abnormalities A) and abnormality images A-IM1, A-IM2 consisting of the at least one nabnormality A only. This decomposition is performed for both images IM1, IM2.

Then, the abnormality images A-IM1, A-IM2 are used to quantify the progression of the abnormality at step S70 by comparing these two, e.g., by correlation. In order to make the comparison of the two abnormality images A-IM1, A-IM2 as accurate as possible, the abnormalities A must be in the same scale (e.g., if the magnification in the first image IM1 is larger than in the second image IM2, the lesion might be wrongly classified as enlarged, while in reality it remained the same size). This is achieved by a registration step S61 on the normal images N-IM1, N-IM2 (alternatively also the original input images IM1, IM2 can be used for registration). The registration step S61 computes a mapping or registration IR1 from the image space of the first medical IM1 to the image space of the second medical image IM2 (e.g., in terms of rigid registration parameters, alternatively in terms of a deformation field DF1 for non-rigid registration). The registration IR1 (the deformation field DF1) is then applied on the first abnormality image A-IM1 to generate a transformed first abnormality image A-IM1-T (step S62). Alternatively, the registration IR1 (the deformation field DF1) may also be applied on the second abnormality image A-IM2 to generate a transformed second abnormality image. The result respectively is one abnormality image in the coordinate system of the respective other abnormality image. This ensures the same scale of the abnormality representations and an accurate disease progression score.

The change CA (optionally in the form of a disease progression score) is then calculated from the abnormality images AIM1, A-IM2 after transformation into a common coordinate system at step S70. Then, each abnormality image A-IM1, A-IM2 corresponds to a certain timestamp. In many cases, the size of the abnormality A could be already a sufficiently good classifier of the change CA. Then simple metrics like correlation or DICE (after binarization) could be a sufficiently good measure.

Alternatively, the mapping could be learned by another network, which is trained using expert rating of disease progression. Another alternative could be a non-rigid registration step S72 of one transformed abnormality image A-IM1-T towards the other abnormality image A-IM2 to generate a second image registration IR2. This could be realized by a deformation field-mapping (deformation field DF2), where each pixel is assigned a vector that defines, where the respective pixel is mapped from one image to the other. Then, the average magnitude and orientation of those vectors could be the measure for disease progression (0 if identical, large positive numbers for large grow, large negative numbers for large shrinkage). Another measure for disease can be the number of pixels which are mapped from the first image to one pixel in the second image. The more pixels are mapped to one pixel in the second image, the smaller is the grow and vice versa.

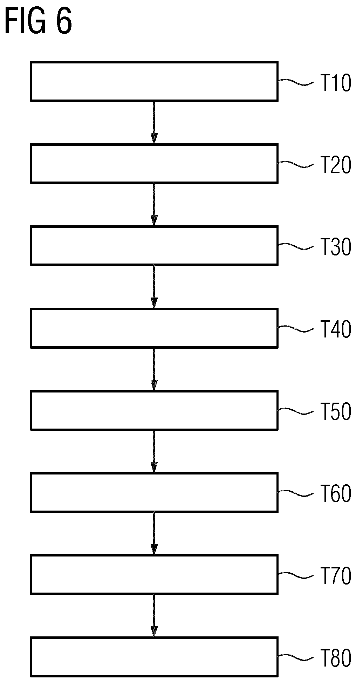

**Figure 6** depicts a schematic representation of the decomposition function TF according to an embodiment. The decomposition function TF according to this embodiment is trained to separate image data relating to abnormal tissue (abnormalities A) and image data relating to normal tissue, in particular, from a chest X-ray image. The generation of a normal image (N-IM1, N-IM2, T-N-IM) is realized with an accordingly trained generator structure GEN that can generate a normal image N-IM1, N-IM2, T-N-IM using the acquired/original medical image IM1, IM2, T-IM as input.

Since images showing the abnormal tissue only are difficult to obtain (for training) it is proposed to use a conditional Generative Adversarial Network (cGAN) approach to train the generator GEN. The cGAN can be trained using real medical images T-IM only. Those medical images T-IM used during training correspond to the first and second medical images IM1, IM2 the decomposition function TF will see when deployed. The training medical images T-IM may or may not comprise abnormalities A. To further increase the efficiency of the training and provide a better decomposition function TF, the fact whether or not a particular training medical image T-IM comprises an abnormality A can be input in the form of a label L. According to some examples the label L may also comprise a location of the abnormality A in the training medical image T-IM.

The training medical image T-IM is input into the generator GEN. This generator GEN learns to derive, from the training medical image T-IM, an image with normal tissue structures only - the training normal image T-N-IM. If the training medical image T-IM does not contain any abnormalities A, the generator GEN is of course supposed to give back essentially the training medical image T-IM as the training normal image T-N-IM.

Once the training normal image T-N-IM has been provided by the generator GEN, the training abnormality image T-A-IM may then be obtained by the difference image between the training normal image T-N-IM and the input training medical image T-IM.

In the training phase of the decomposition function TF, the generator GEN according to some examples is trained by the feedback from a discriminator DIS. The discriminator DIS simultaneously learns to discriminate "real" normal medical images (that is, medical images not depicting any abnormalities A in the first place) from the normal images T-N-IM synthetically generated by the generator GEN. In turn, the generator GEN tries to generate normal images T-N-IM that are accepted by the discriminator DIS, while the discriminator DIS tries to detect those synthetically generated images as "fake-normal"-images. By training both together, the generator GEN learns to project training medical images T-IM with abnormalities A to an image space of hypothetical normal images not comprising any abnormalities A.

The generator GEN is trained based on the feedback from the discriminator DIS. Specifically, the feedback from the discriminator DIS can be used as a first loss function LF1 to adjust the generator GEN. This first loss function LF1 may be denoted as an adversarial loss.

In addition to the adversarial loss by way of the first loss function LF1, the generator GEN may be trained based on the appearance of the abnormality image T-A-IM. Based on a quantification of the structure of the abnormality image T-A-IM, a second loss function LF2 can be defined. According to some embodiments, the second loss function LF2 may be based on a sparsity loss in terms of Total Variation (TV). If abnormal images are available, also an image loss between the ground-truth and training abnormality images T-A-IM can be used according to other examples.

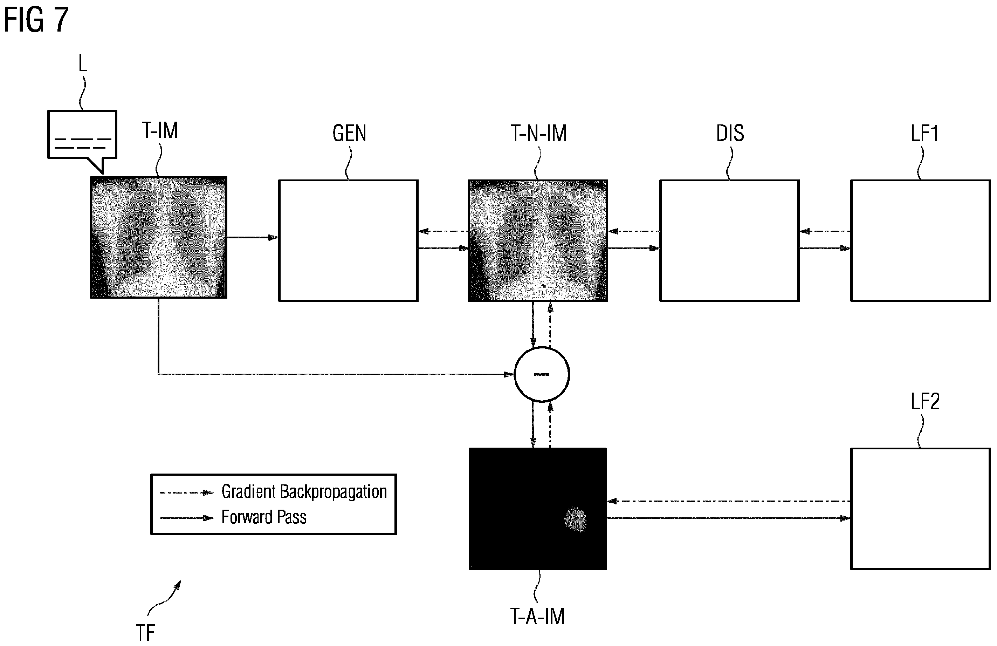

**Figure 7** depicts a method for providing a decomposition function TF to decompose a medical image T-IM depicting at least one abnormality A in a body part of a patient into a synthetically generated normal image T-N-IM showing the body part without any abnormalities A and an abnormality image TA-IM showing the abnormalities A only. The method comprises several steps. The order of the steps does not necessarily correspond to the numbering of the steps but may also vary between different embodiments of the present invention.

A first step T10 is directed to provide a plurality of training medical images T-IM. The training medical images T-IM are preferably of the same type as the medical images IM1, IM2 to be processed by the deployed and readily trained machine learned model TF. Accordingly, the training medical images T-IM each likewise show a body part of a patient with or without abnormalities A.

In a next step T20, the training medical images T-IM are input into the generator part GEN of the decomposition function TF. That followed, a training normal image T-N-IM is obtained as output from the generator GEN in step T30.

In step T40, the training normal image T-N-IM is input into the discriminator part DIS of the decomposition function TF. The discriminator DIS has been trained to discriminate "real" normal images that did not comprise any abnormalities A in the first place from synthetically generated training normal images T-N-IM from the generator GEN. The evaluation result of the discriminator DIS may be used calculate a first loss, the so-called adversarial loss, by way of a first loss function LF1 (step T50).

In optional step T60, a training abnormality image T-A-IM may be generated, e.g., by subtracting the training normal image T-N-IM from the training image T-IM or vice versa. In optional step T70, the training abnormality image T-A-IM may be used to determine a second loss by way of a second loss function LF2. One way of implementing this would be comparing training abnormality images T-A-IM with verified abnormality images which have been positively reviewed by a human or have been manually generated by a human. Another way of implementing the second loss function may be as a sparsity loss in terms of Total Variation (TV).

At step T80, first and second losses are used to adjust the decomposition function TF. That followed, the steps of generating training normal images T-N-IM, training abnormality images T-A-IM, determining first and/or second losses are repeated with further training medical images T-IM until the decomposition function TF is able to generate results that are acceptable (i.e., until local minima of the loss functions LF1, LF2 are reached).

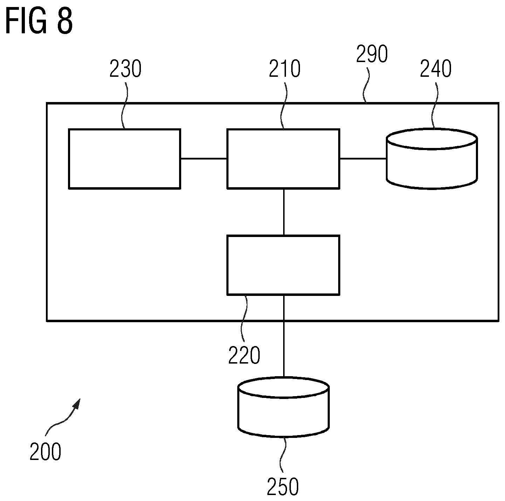

**Figure 8** illustrates an embodiment of a system 200 for training a trained function TF. The system comprises a processor 210, an interface 220, a memory 230, a storage 240, and a database 250. Processor 210, interface 220, memory 230 and storage 240 may be embodied by a computer 290. Processor 210 controls the overall operation of the computer 200 by executing computer program instructions which define such operation. The computer program instructions may be stored in memory 230 or in storage 240 and loaded into memory 230 when execution of the computer program instructions is desired. Storage 240 may be a local storage as a component of the system 200, or a remote storage accessible over a network, such as a component of a server or cloud system. The method steps illustrated in Figure 10 may be defined by the computer program instructions stored in memory 230 and/or storage 240, and controlled by processor 210 executing the computer program instructions.

Database 250 is a storage device such a cloud or local storage serving as an archive for the training data sets comprising medical images T-IM and labels L as introduced above. Database 250 may be connected to computer 290 for receipt of one or more medical images. It is also possible to implement database 250 and computer 290 as a single device. It is further possible that database 250 and computer 290 communicate wirelessly or with wired connection through a network. Interface 220 is configured to interact with database 250.

Wherever meaningful, individual embodiments or their individual aspects and features can be combined or exchanged with one another without limiting or widening the scope of the present invention. Advantages which are described with respect to one embodiment of the present invention are, wherever applicable, also advantageous to other embodiments of the present invention.

## Claims

1. Computer-implemented method for determining a change (CA) of an abnormality (A) in an anatomical region of a patient, the method comprising:
- receiving (S10) a first medical image (IM1) of an anatomical region of a patient, the first medical image (IM1) being acquired at a first instance of time and depicting at least one abnormality (A) in the anatomical region;
- receiving (S20) a second medical image (IM2) of the anatomical region of the patient, the second medical image (IM2) being acquired at a second instance of time;
- providing (S30) a decomposition function (TF) configured to extract, from a medical image of an anatomical region with one or more abnormalities, an abnormality image only depicting the image regions of the medical image of the one or more abnormalities, wherein the decomposition function (TF) comprises an inpainting function configured to inpaint abnormalities (A) within the medical image (IM1, IM2) to generate a normal image (N-IM1, N-IM2) of the medical image (IM1, IM2), and the decomposition function (TF) is further configured to extract the abnormality image (A-IM1, A-IM2) from the medical image (IM1, IM2) by subtracting the generated normal image (N-IM1, N-IM2) from the medical image (IM1, IM2) or vice versa;
- generating (S40) a first abnormality image (A-IM1) of the first medical image (IM1) by applying the decomposition function (TF) to the first medical image (IM1);
- generating (S50) a second abnormality image (A-IM2) of the second medical image (IM2) by applying the decomposition function (TF) to the second medical image (IM2);
- comparing (S60) the first abnormality image (A-IM1) and the second abnormality image (A-IM2);
- determining (S70) a change (CA) of the at least one abnormality (A) based on the step (S60) of comparing.

2. Method according to claim 1,
- the decomposition function (TF) being further configured to extract, from medical images of anatomical regions with one or more abnormalities, a normal image of the anatomical region not depicting the one or more abnormalities; and
the method further comprising the steps:
- generating (S40) a first normal image (N-IM1) of the first medical image (IM1) by applying the decomposition function (TF) to the first medical image (IM1); and
- generating (S50) a second normal image (N-IM2) of the second medical image (IM2) by applying the decomposition function (TF) to the second medical image (IM2).

3. Method according to any of claims 1 or 2, wherein:
- the step of comparing (S60) comprises: determining (S61) at least one image registration (IR1, IR2) between the image space of the first abnormality image (A-IM1) and the image space of the second abnormality image (A-IM2); and
- the step of determining (S70) a change (CA) of the at least one abnormality (A) is based on the at least one image registration (IR1, IR2).

4. Method according to claim 3, wherein:
- the at least one image registration (IR1) is determined by registering the first medical image (IM1) with the second medical image (IM2).

5. Method according to claim 3 in combination with claim 2, wherein:
- the at least one image registration (IR1) is determined by registering the first normal image (N-IM1) with the second normal image (N-IM2).

6. Method according to any one of claims 4 or 5, the method further comprising:
- calculating (S71) a deformation field (DF2) based on the at least one image registration (IR1, IR2), the deformation field (DF2) being suited to map an image region of the at least one abnormality (A) in the first abnormality (A-IM1) image to a corresponding image region of the at least one abnormality (A) in the second abnormality image (A-IM2);
- wherein the change (CA) is determined (S73) based on the deformation field (DF2).

7. Method according to any one of the preceding claims, wherein:
- the step of determining (S70) a change (CA) comprises calculating a score measuring the size change of the at least one abnormality (A) from the first instance of time to the second instance of time.

8. Method according to any one of the preceding claims, wherein:
- the decomposition function (TF) comprises a trained function, the trained function in particular comprising a conditional Generative Adversarial Network (cGAN).

9. Method according to any one of the preceding claims, further comprising:
- providing (S80), in particular to a user via a user interface (10), the determined change (CA), in particular, in the form of an assistance image (AI) based on the first and/or second medical image (IM1, IM2) with the at least one abnormality (A) and/or the change (CA) being highlighted.

10. Method according to any one of the preceding claims, wherein:
- the anatomical region comprises the lung of the patient; and
- the at least one abnormality (A) comprises a lung lesion in the lung of the patient, the lung lesion in particular comprising any one of: a lung nodule, a consolidation, or an emphysema.

11. Method according to any one of the preceding claims, wherein:
- first and second medical images (IM1, IM2) are X-ray images of the chest of the patient.

12. System (1) for determining a change (CA) of an abnormality (A) in an anatomical region of a patient comprising an interface unit (10, 26) and a computing unit (30),
the interface unit (10, 26) being configured to:
- receive (S10) a first medical image (IM1) of an anatomical region of a patient, the first medical image (IM1) being acquired at a first instance of time and depicting at least one abnormality (A) in the anatomical region; and
- receive (S20) a second medical image (IM2) of the anatomical region of the patient, the second medical (IM2) image being acquired at a second instance of time; and
the computing unit (30) being configured to:
- provide (S30) a decomposition function (TF) configured to extract, from a medical image (IM1, IM2) of an anatomical region with one or more abnormalities (A), an abnormality image (A-IM1, A-IM2) only depicting the one or more abnormalities (A), wherein the decomposition function (TF) comprises an inpainting function configured to inpaint abnormalities (A) within the medical image (IM1, IM2) to generate a normal image (N-IM1, N-IM2) of the medical image (IM1, IM2), and the decomposition function (TF) is further configured to extract the abnormality image (A-IM1, A-IM2) from the medical image (IM1, IM2) by subtracting the generated normal image (N-IM1, N-IM2) from the medical image (IM1, IM2) or vice versa;
- generate (S40) a first abnormality image (A-IM1) of the first medical image (IM1) by applying the decomposition function (TF) to the first medical image (IM1);
- generate (S50) a second abnormality image (A-IM2) of the second medical image (IM2) by applying the decomposition function (TF) to the second medical image (IM2);
- compare (S60) the first abnormality image (A-IM1) and the second abnormality image (A-IM2); and
- determine (S70) a change (CA) of the at least one abnormality (A) based on the comparison (S60) of the first abnormality image (A-IM1) and the second abnormality image (A-IM2).

13. Computer program product comprising program elements which induce a computing unit (30) of a system (1) for determining a change (CA) of an abnormality (A) in an anatomical region of a patient to perform the steps according to the method of any of claims 1 to 11, when the program elements are loaded into a memory of the computing unit (30).

14. Computer-readable medium on which program elements are stored that are readable and executable by a computing unit (30) of a system (1) for determining a change (CA) of an abnormality (A) in an anatomical region of a patient to perform steps of the method according to any of claims 1 to 11, when the program elements are executed by the computing unit (30).

## Patentansprüche

1. Computerimplementiertes Verfahren zum Bestimmen einer Änderung (CA) einer Anomalie (A) in einem anatomischen Bereich eines Patienten, wobei das Verfahren Folgendes umfasst:
- Empfangen (S10) eines ersten medizinischen Bildes (IM1) eines anatomischen Bereichs eines Patienten, wobei das erste medizinische Bild (IM1) zu einem ersten Zeitpunkt erfasst wird und mindestens eine Anomalie (A) in dem anatomischen Bereich darstellt;
- Empfangen (S20) eines zweiten medizinischen Bildes (IM2) des anatomischen Bereichs des Patienten, wobei das zweite medizinische Bild (IM2) zu einem zweiten Zeitpunkt erfasst wird;
- Bereitstellen (S30) einer Zerlegungsfunktion (TF), die dazu ausgelegt ist, aus einem medizinischen Bild eines anatomischen Bereichs mit einer oder mehreren Anomalien ein Anomaliebild zu extrahieren, das nur die Bildbereiche des medizinischen Bildes der einen oder mehreren Anomalien darstellt, wobei die Zerlegungsfunktion (TF) eine Inpainting-Funktion umfasst, die dazu ausgelegt ist, Anomalien (A) innerhalb des medizinischen Bildes (IM1, IM2) zu retuschieren, um ein normales Bild (N-IM1, N-IM2) des medizinischen Bildes (IM1, IM2) zu erzeugen, und die Zerlegungsfunktion (TF) ferner dazu ausgelegt ist, das Anomaliebild (A-IM1, A-IM2) aus dem medizinischen Bild (IM1, IM2) durch Subtrahieren des erzeugten normalen Bildes (N-IM1, N-IM2) von dem medizinischen Bild (IM1, IM2) oder umgekehrt zu extrahieren;
- Erzeugen (S40) eines ersten Anomaliebildes (A-IM1) des ersten medizinischen Bildes (IM1) durch Anwenden der Zerlegungsfunktion (TF) auf das erste medizinische Bild (IM1);
- Erzeugen (S50) eines zweiten Anomaliebildes (A-IM2) des zweiten medizinischen Bildes (IM2) durch Anwenden der Zerlegungsfunktion (TF) auf das zweite medizinische Bild (IM2);
- Vergleichen (S60) des ersten Anomaliebildes (A-IM1) und des zweiten Anomaliebildes (A-IM2);
- Bestimmen (S70) einer Änderung (CA) der mindestens einen Anomalie (A) basierend auf dem Schritt (S60) des Vergleichens.

2. Verfahren nach Anspruch 1,
- wobei die Zerlegungsfunktion (TF) ferner dazu ausgelegt ist, aus medizinischen Bildern von anatomischen Bereichen mit einer oder mehreren Anomalien ein normales Bild des anatomischen Bereichs zu extrahieren, das die eine oder die mehreren Anomalien nicht darstellt; und wobei das Verfahren ferner die folgenden Schritte umfasst:
- Erzeugen (S40) eines ersten normalen Bildes (N-IM1) des ersten medizinischen Bildes (IM1) durch Anwenden der Zerlegungsfunktion (TF) auf das erste medizinische Bild (IM1); und
- Erzeugen (S50) eines zweiten normalen Bildes (N-IM2) des zweiten medizinischen Bildes (IM2) durch Anwenden der Zerlegungsfunktion (TF) auf das zweite medizinische Bild (IM2).

3. Verfahren nach einem der Ansprüche 1 oder 2, wobei:
- der Schritt des Vergleichens (S60) Folgendes umfasst: Bestimmen (S61) mindestens einer Bildregistrierung (IR1, IR2) zwischen dem Bildraum des ersten Anomaliebildes (A-IM1) und dem Bildraum des zweiten Anomaliebildes (A-IM2); und
- der Schritt des Bestimmens (S70) einer Änderung (CA) der mindestens einen Anomalie (A) auf der mindestens einen Bildregistrierung (IR1, IR2) basiert.

4. Verfahren nach Anspruch 3, wobei:
- die mindestens eine Bildregistrierung (IR1) durch Registrieren des ersten medizinischen Bildes (IM1) mit dem zweiten medizinischen Bild (IM2) bestimmt wird.

5. Verfahren nach Anspruch 3 in Kombination mit Anspruch 2, wobei:
- die mindestens eine Bildregistrierung (IR1) durch Registrieren des ersten normalen Bildes (N-IM1) mit dem zweiten normalen Bild (N-IM2) bestimmt wird.

6. Verfahren nach einem der Ansprüche 4 oder 5, wobei das Verfahren ferner Folgendes umfasst:
- Berechnen (S71) eines Deformationsfeldes (DF2) basierend auf der mindestens einen Bildregistrierung (IR1, IR2), wobei das Deformationsfeld (DF2) dazu geeignet ist, einen Bildbereich der mindestens einen Anomalie (A) in dem ersten Anomaliebild (A-IM1) auf einen entsprechenden Bildbereich der mindestens einen Anomalie (A) in dem zweiten Anomaliebild (A-IM2) abzubilden;
- wobei die Änderung (CA) basierend auf dem Deformationsfeld (DF2) bestimmt (S73) wird.

7. Verfahren nach einem der vorhergehenden Ansprüche, wobei:
- der Schritt des Bestimmens (S70) einer Änderung (CA) Berechnen einer Bewertung umfasst, die die Größenänderung der mindestens einen Anomalie (A) vom ersten Zeitpunkt zum zweiten Zeitpunkt misst.

8. Verfahren nach einem der vorhergehenden Ansprüche, wobei:
- die Zerlegungsfunktion (TF) eine trainierte Funktion umfasst, wobei die trainierte Funktion insbesondere ein conditional Generative Adversarial Network (cGAN) umfasst.

9. Verfahren nach einem der vorhergehenden Ansprüche, ferner umfassend:
- Bereitstellen (S80), insbesondere zu einem Benutzer über eine Benutzeroberfläche (10), der bestimmten Änderung (CA), insbesondere in Form eines Assistenzbildes (AI) basierend auf dem ersten und/oder zweiten medizinischen Bild (IM1, IM2), wobei die mindestens eine Anomalie (A) und/oder die Änderung (CA) hervorgehoben sind.

10. Verfahren nach einem der vorhergehenden Ansprüche, wobei:
- der anatomische Bereich die Lunge des Patienten umfasst; und
- die mindestens eine Anomalie (A) eine Lungenläsion in der Lunge des Patienten umfasst, wobei die Lungenläsion insbesondere eines von Folgendem umfasst: einen Lungenknoten, eine Lungenverdichtung oder ein Emphysem.

11. Verfahren nach einem der vorhergehenden Ansprüche, wobei:
- erste und zweite medizinische Bilder (IM1, IM2) Röntgenbilder des Thorax des Patienten sind.

12. System (1) zum Bestimmen einer Änderung (CA) einer Anomalie (A) in einem anatomischen Bereich eines Patienten, umfassend eine Schnittstelleneinheit (10, 26) und eine Recheneinheit (30), wobei die Schnittstelleneinheit (10, 26) zu Folgendem ausgelegt ist:
- Empfangen (S10) eines ersten medizinischen Bildes (IM1) eines anatomischen Bereichs eines Patienten, wobei das erste medizinische Bild (IM1) zu einem ersten Zeitpunkt erfasst wird und mindestens eine Anomalie (A) in dem anatomischen Bereich darstellt; und
- Empfangen (S20) eines zweiten medizinischen Bildes (IM2) des anatomischen Bereichs des Patienten, wobei das zweite medizinische Bild (IM2) zu einem zweiten Zeitpunkt erfasst wird; und
wobei die Recheneinheit (30) zu Folgendem ausgelegt ist:
- Bereitstellen (S30) einer Zerlegungsfunktion (TF), die dazu ausgelegt ist, aus einem medizinischen Bild (IM1, IM2) eines anatomischen Bereichs mit einer oder mehreren Anomalien (A) ein Anomaliebild (A-IM1, A-IM2) zu extrahieren, das nur die eine oder die mehreren Anomalien (A) darstellt, wobei die Zerlegungsfunktion (TF) eine Inpainting-Funktion umfasst, die dazu ausgelegt ist, Anomalien (A) innerhalb des medizinischen Bildes (IM1, IM2) zu retuschieren, um ein normales Bild (N-IM1, N-IM2) des medizinischen Bildes (IM1, IM2) zu erzeugen, und die Zerlegungsfunktion (TF) ferner dazu ausgelegt ist, das Anomaliebild (A-IM1, A-IM2) aus dem medizinischen Bild (IM1, IM2) durch Subtrahieren des erzeugten normalen Bildes (N-IM1, N-IM2) von dem medizinischen Bild (IM1, IM2) oder umgekehrt zu extrahieren;
- Erzeugen (S40) eines ersten Anomaliebildes (A-IM1) des ersten medizinischen Bildes (IM1) durch Anwenden der Zerlegungsfunktion (TF) auf das erste medizinische Bild (IM1);
- Erzeugen (S50) eines zweiten Anomaliebildes (A-IM2) des zweiten medizinischen Bildes (IM2) durch Anwenden der Zerlegungsfunktion (TF) auf das zweite medizinische Bild (IM2);
- Vergleichen (S60) des ersten Anomaliebildes (A-IM1) und des zweiten Anomaliebildes (A-IM2); und
- Bestimmen (S70) einer Änderung (CA) der mindestens einen Anomalie (A) basierend auf dem Vergleich (S60) des ersten Anomaliebildes (A-IM1) und des zweiten Anomaliebildes (A-IM2).

13. Computerprogrammprodukt, das Programmelemente umfasst, die eine Recheneinheit (30) eines Systems (1) zum Bestimmen einer Änderung (CA) einer Anomalie (A) in einem anatomischen Bereich eines Patienten veranlassen, um die Schritte gemäß dem Verfahren nach einem der Ansprüche 1 bis 11 durchzuführen, wenn die Programmelemente in einen Speicher der Recheneinheit (30) geladen werden.

14. Computerlesbares Medium, auf dem Programmelemente gespeichert sind, die durch eine Recheneinheit (30) eines Systems (1) zum Bestimmen einer Änderung (CA) einer Anomalie (A) in einem anatomischen Bereich eines Patienten auslesbar und ausführbar sind, um Schritte des Verfahrens nach einem der Ansprüche 1 bis 11 durchzuführen, wenn die Programmelemente durch die Recheneinheit (30) ausgeführt werden.

## Revendications

1. Procédé mis en œuvre par ordinateur de détermination du changement (CA) d'une anomalie (A) dans une région anatomique d'un patient, le procédé comprenant :
- recevoir (S10) une première image (IM1) médicale d'une région anatomique d'un patient, la première image (IM1) médicale étant acquise à une première instance de temps et dépeignant au moins une anomalie (A) dans la région anatomique ;
- recevoir (S20) une deuxième image (IM2) médicale de la région anatomique du patient, la deuxième image (IM2) médicale étant acquise à une deuxième instance de temps ;
- se procurer (S30) une fonction (TF) de décomposition configurée pour extraire, d'une image médicale d'une région anatomique ayant une ou plusieurs anomalies, une image d'anomalie dépeignant seulement les régions de l'image médicale de la une ou des plusieurs anomalies, dans lequel la fonction (TF) de décomposition comprend une fonction ne dépeignant pas configurée pour ne pas dépeindre des anomalies (A) dans l'image (IM1, IM2) médicale, afin de créer une image (N-IM1, N-IM2) normale de l'image (IM1, IM2) médicale, et la fonction (TF) de décomposition étant configurée en outre pour extraire l'image (A-IM1, A-IM2) d'anomalie de l'image (IM1, IM2) médicale en soustrayant l'image (N-IM1, N-IM2) normale créée de l'image (IM1, IM2) médicale ou vice versa ;
- créer (S40) une première image (A-IM1) d'anomalie de la première image (IM1) médicale en appliquant la fonction (TF) de décomposition à la première image (IM1) médicale ;
- créer (S50) une deuxième image (A-IM2) d'anomalie de la deuxième image (IM2) médicale en appliquant la fonction (TF) de décomposition à la deuxième image (IM2) médicale ;
- comparer (S60) la première image (A-IM1) d'anomalie et la deuxième image (A-IM2) d'anomalie ;
- déterminer (S70) un changement (CA) de la au moins une anomalie (A) sur la base du stade (S60) de comparaison.

2. Procédé suivant la revendication 1,
- la fonction (TF) de décomposition étant configurée en outre pour extraire, d'images médicales de régions anatomiques ayant une ou plusieurs anomalies, une image normale de la région anatomique ne dépeignant pas la une ou les plusieurs anomalies ; et le procédé comprenant en outre les stades :
- créer (S40) une première image (N-IM1) normale de la première image (IM1) médicale en appliquant la fonction (TF) de décomposition à la première image (IM1) médicale ; et
- créer (S50) une deuxième image (N-IM2) normale de la deuxième image (IM2) médicale en appliquant la fonction (TF) de décomposition à la deuxième image (IM2) médicale.

3. Procédé suivant l'une quelconque des revendications 1 ou 2, dans lequel :
- le stade de comparaison (S60) comprend : déterminer (S61) au moins une correspondance (IR1, IR2) d'image entre l'espace d'image de la première image (A-IM1) d'anomalie et l'espace d'image de la deuxième image (A-IM2) d'anomalie ; et
- le stade de détermination (S70) d'un changement (CA) de la au moins une anomalie (A) repose sur la au moins une correspondance (IR1, IR2) d'image.

4. Procédé suivant la revendication 3, dans lequel :
- la au moins une correspondance (IR1) d'image est déterminée en faisant correspondre la première image (IM1) médicale à la deuxième image (IM2) médicale.

5. Procédé suivant la revendication 3 en combinaison avec la revendication 2, dans lequel :
- la au moins une correspondance (IR1) d'image est déterminée en faisant correspondre la première image (N-IM1) normale à la deuxième image (N-IM2) normale.

6. Procédé suivant l'une quelconque des revendications 4 ou 5, le procédé comprenant en outre :
- calculer (S71) un champ (DF2) de déformation sur la base de la au moins une correspondance (IR1, IR2) d'image, le champ (DF2) de déformation étant propre à faire correspondre une région d'image de la au moins une anomalie (A) dans la première image d'anomalie (A-IM1) à une région d'image correspondante de la au moins une anomalie (A) dans la deuxième image (A-IM2) d'anomalie ;
- dans lequel le changement (CA) est déterminé (S73) sur la base du champ (DF2) de déformation.

7. Procédé suivant l'une quelconque des revendications précédentes, dans lequel :
- le stade de détermination (S70) d'un changement (CA) comprend calculer un score mesurant le changement de dimension de la au moins une anomalie (A) de la première instance de temps à la deuxième instance de temps.

8. Procédé suivant l'une quelconque des revendications précédentes, dans lequel :
- la fonction (TF) de décomposition comprend une fonction ayant subi un apprentissage, la fonction ayant subi un apprentissage comprenant, en particulier un conditional generative adversarial network (cGAN).

9. Procédé suivant l'une quelconque des revendications précédentes, comprenant en outre :
- donner (S80), en particulier à un utilisateur par l'intermédiaire d'une interface (10) d'utilisateur, le changement (CA) déterminé, en particulier sous la forme d'une image (AI) d'assistance reposant sur la première et/ou la deuxième image (IM1, IM2) médicale ayant la au moins une anomalie (A) et/ou le changement (CA) étant mis en lumière.

10. Procédé suivant l'une quelconque des revendications précédentes, dans lequel :
- la région anatomique comprend le poumon du patient ; et
- la au moins une anomalie (A) comprend une lésion du poumon du patient, la lésion du poumon comprenant, en particulier l'un d'un : nodule pulmonaire, une consolidation ou un emphysème.

11. Procédé suivant l'une quelconque des revendications précédentes, dans lequel :
- des première et deuxième images (IM1, IM2) médicales sont des images aux rayons X du thorax du patient.

12. Système (1) de détermination du changement (CA), d'une anomalie (A) dans une région anatomique d'un patient comprenant une unité (10, 26) d'interface et une unité (30) informatique, l'unité (10, 26) d'interface étant configurée pour :
- recevoir (S10) une première image (IM1) médicale d'une région anatomique d'un patient, la première image (IM1) médicale étant acquise à une première instance de temps et dépeignant au moins une anomalie (A) dans la région anatomique ; et
- recevoir (S20) une deuxième image (IM2) médicale de la région anatomique du patient, la deuxième image (IM2) médicale étant acquise à une deuxième instance de temps ; et
l'unité (30) informatique étant configurée pour :
- donner (S30) une fonction (TF) de décomposition configurée pour extraire, d'une image (IM1, IM2) médicale d'une région anatomique ayant une ou plusieurs anomalies (A), une image (A-IM1, A-IM2) d'anomalie dépeignant seulement les régions de l'image médicale de la une ou des plusieurs anomalies (A), dans lequel la fonction (TF) de décomposition comprend une fonction ne dépeignant pas configurée pour ne pas dépeindre des anomalies (A) dans l'image (IM1, IM2) médicale, afin de créer une image (N-IM1, N-IM2) normale de l'image (IM1, IM2) médicale, et la fonction (TF) de décomposition étant configurée en outre pour extraire l'image (A-IM1, A-IM2) d'anomalie de l'image (IM1, IM2) médicale en soustrayant l'image (N-IM1, N-IM2) normale créée de l'image (IM1, IM2) médicale ou vice versa ;
- créer (S40) une première image (A-IM1) d'anomalie de la première image (IM1) médicale en appliquant la fonction (TF) de décomposition à la première image (IM1) médicale ;
- créer (S50) une deuxième image (A-IM2) d'anomalie de la deuxième image (IM2) médicale en appliquant la fonction (TF) de décomposition à la deuxième image (IM2) médicale ;
- comparer (S60) la première image (A-IM1) d'anomalie et la deuxième image (A-IM2) d'anomalie ; et
- déterminer (S70) un changement (CA) de la au moins une anomalie (A) sur la base de la comparaison (S60) de la première image (A-IM1) d'anomalie et de la deuxième image (A-IM2) d'anomalie.

13. Produit de programme d'ordinateur comprenant des éléments de programme, qui font qu'une unité (30) informatique d'un système (1) de détermination d'un changement (CA) d'une anomalie (A) dans une région anatomique d'un patient exécute les stades suivants le procédé de l'une quelconque des revendications 1 à 11, lorsque les éléments de programme sont chargés dans une mémoire de l'unité (30) informatique.

14. Support déchiffrable par ordinateur, sur lequel sont mis en mémoire des éléments de programme, qui peuvent être déchiffrés et exécutés par une unité (30) informatique d'un système (1) de détermination d'un changement (CA) d'une anomalie (A) dans une région anatomique d'un patient pour effectuer des stades du procédé suivant l'une quelconque des revendications 1 à 11, lorsque les éléments de programme sont exécutés par l'unité (30) informatique.
